# EUROPEAN PATENT SPECIFICATION

(11) **EP 3 727 391 B1**
(45) Date of publication and mention of the grant of the patent: **23.09.2026**
(21) Application number: 18833541.8
(22) Date of filing: 19.12.2018
(51) Int. Cl.: A61K 31/519, A61K 31/522, A61P 35/00

(54) **PHARMACEUTICAL COMPOSITION FOR USE IN THE TREATMENT OF CANCER**
PHARMAZEUTISCHE ZUSAMMENSETZUNG ZUR VERWENDUNG IN DER BEHANDLUNG VON KREBS
COMPOSITION PHARMACEUTIQUE POUR L'UTILISATION DANS LE TRAITEMENT DU CANCER

(30) Priority: 19.12.2017 IN 201721045725; 19.12.2017 IN 201721045726; 19.12.2017 IN 201721045727
(43) Date of publication of application: 28.10.2020
(73) Proprietor: Impetis Biosciences Ltd., Mumbai City, Maharashtra 400001 (IN)
(72) Inventor: MOOKHTIAR, Kasim, Mumbai 400001 (IN); BASU, Sujay, Mumbai 400001 (IN); ROUDURI, Sreekanth, Mumbai 400001 (IN); MERU, Ashwin, Mumbai 400001 (IN)
(74) Representative: Stepney, Gregory John
(86) International application number: PCT/IN2018/050859
(87) International publication number: WO 2019/123482

(56) References cited:
- WO-A1-2012/035548
- WO-A2-2010/103547
- WO-A2-2012/038980
- FRANCO R. ET AL.: "Adenosine Receptor Antagonists to Combat Cancer and to Boost Anti-Cancer Chemotherapy and Immunotherapy", CELLS, vol. 10, 2021, pages 2831 - 2844
- Cyncado Therapeutics - Confidential Investor Presentation
- DAVID ALLARD ET AL: "Targeting A2 adenosine receptors in cancer", IMMUNOLOGY AND CELL BIOLOGY, vol. 95, no. 4, 21 February 2017 (2017-02-21), AU, pages 333 - 339, XP055557743, ISSN: 0818-9641, DOI: 10.1038/icb.2017.8
- DIPTI VIJAYAN ET AL: "Targeting immunosuppressive adenosine in cancer", NATURE REVIEWS. CANCER, vol. 17, no. 12, 23 October 2017 (2017-10-23), GB, pages 709 - 724, XP055557876, ISSN: 1474-175X, DOI: 10.1038/nrc.2017.86

## Description

### FIELD OF THE INVENTION

The present invention relates to a pharmaceutical composition comprising 5-amino-3-[2-[4-[2-fluoro-4-(2-methoxyethoxy)phenyl]piperazin-1-yl]ethyl]-8-(2-furyl)-1-methyl-[1,2,4]triazolo[5,1-f]purin-2-one **(32)** or a pharmaceutically acceptable salt thereof for use in the treatment of a condition or disorder selected from breast cancer and colon cancer.

### BACKGROUND OF INVENTION

Adenosine is an endogenous modulator of a wide range of physiological functions and is implicated in several pathologies. Recent advances in molecular biology coupled with several pharmacological studies have lead to identification of at least four subtypes of adenosine receptors, A₁, A_{2A}, A_{2B} and A₃. The A₁ and A₃ receptors down-regulate cellular cAMP levels through their coupling to G protein, which inhibit adenylate cyclase. In contrast, A_{2A} and A_{2B} receptors couple to G protein that activate adenylate cyclase and increase intracellular levels of cAMP.

Advances in understanding the role of adenosine and its receptors in physiology and pathophysiology as well as new developments in medicinal chemistry of these receptors have identified potential therapeutic areas for drug development. With the combination of pharmacological data using selective ligands and genetically modified mice, important progress has been made towards understanding of the role of adenosine receptors (Ars) in a variety of diseases, such as inflammatory conditions, sepsis, heart attack, ischemia-reperfusion injury, vascular injury, spinal cord injury, chronic obstructive pulmonary disease (COPD), asthma, diabetes, obesity, inflammatory bowel disease, retinopathy, and Parkinson's Disease (PD).

In the central nervous system, A_{2A} antagonists can have antidepressant properties and stimulate cognitive functions. Epidemiological evidence shows a protective role for caffeine in Parkinson's disease. Moreover, A_{2A} receptor density is found to be very high in the basal ganglia which regulate motor control function. Hence, selective A_{2A} antagonists can improve motor impairment due to neurodegenerative diseases such as Parkinson's disease (Trends Pharmacol. Sci. 1997, 18, 338-344), senile dementia as in Alzheimer's disease, psychoses, stroke and be potentially effective in the treatment of cerebral ischaemia (Life Sci. 1994, 55, 61-65). A₂ₐ antagonists may also be employed for the treatment or management of attention related disorders such as attention deficit disorder and attention deficit hyperactivity disorder, extra pyramidal syndrome, e.g., dystonia, akathisia, pseudoparkinsonism and tardive dyskinesia, and disorders of abnormal movement such as restless leg syndrome and periodic limb movement in sleep. Several of these indications have been disclosed in patent applications (eg. WO 02/055083, WO 05/044245 and WO 06/132275). Adenosine A_{2A} antagonists could also be useful in the treatment of amyotrophic lateral sclerosis, cirrhosis, and fibrosis and fatty liver (US2007037033, WO 01/058241). A_{2A} receptor antagonists are also useful for the mitigation of addictive behavior (WO 06/009698) and for the treatment and prevention of dermal fibrosis in diseases such as scleroderma (Arthritis & Rheumatism, 54(8), 2632-2642, 2006).

Parkinson's disease (PD) is a progressive, incurable disorder with no definite preventive treatment, although drugs are available to alleviate the symptoms and/or slow down the progress of the disease. Among the various strategies, A_{2A} AR blockers are considered a potential approach to treatment of the disease. Within the brain A_{2A} ARs are richly expressed in the striatum, nucleus accumbens, and olfactory tubercle. Co-expression of A_{2A} with D2 dopamine receptors has been reported in the GABAergic striatopallidal neurons where adenosine and dopamine agonists exert antagonistic effects in the regulation of locomotor activity. Activation of A_{2A} ARs in striatopallidal neurons decreases the affinity of D2 receptors for dopamine, antagonizing the effects of D2 receptors. The negative interaction between A_{2A} and D2 receptors is at the basis of the use of A_{2A} antagonists as a novel therapeutic approach in the treatment of PD (Pharmacol. Ther. 2005, 105, 267). The recent discovery that the A_{2A} can form functional heteromeric receptor complexes with other Gprotein-coupled receptors such as D2 receptors and the mGlu5 receptors has also suggested new opportunities for the potential of A_{2A} antagonists in PD (J. Mol. Neurosci. 2005, 26, 209).

Adenosine signaling is known to serve apoptotic, angiogenic and proinflammatory functions and might be relevant to the pathogenesis of asthma and chronic obstructive pulmonary disease (Trends in Pharmacological Sciences, 2003, 24, 8). Extracellular adenosine acts as a local modulator with a generally cytoprotective function in the body. Its effects on tissue protection and repair fall into four categories: increasing the ratio of oxygen supply to demand; protecting against ischaemic damage by cell conditioning; triggering anti-inflammatory responses; and the promotion of angiogenesis.

In recent years, A_{2A} receptor has shown exciting progress in the development of immunotherapy for the treatment of cancer (Cancer Immunol Res. 2015, 3, 506-517). Adenosine generation in tumor microenvironment is an active metabolic mechanism used by cancer cells to avoid anti-tumor immunosurveillance and increase metastasis. Ectonucleotidase CD73 and CD39 (highly expressed on tumor cells and stromal cells) convert ATP released by dying tumor cells to adenosine. Adenosine signaling through A_{2A} receptors enhances pro-tumoral responses in the tumor microenvironment contributing to tumor growth and metastases. A_{2A} receptors are expressed on several immune cell types- T lymphocytes, dendritic cells, natural killer cells. A_{2A} receptor activation on T cells and NK cells causes immunosuppression by reducing their proliferation, cytokine production and tumor killing activity. A_{2A} antagonists could induce anti-tumoral responses in multiple types of cancer when used as stand alone or in combination with existing immunotherapies or radiotherapy or chemotherapy. Cancers that could benefit from A_{2A} antagonist therapy include melanoma, triple negative breast cancer, colon cancer, colorectal cancer, lung cancer, prostate cancer, renal cell cancer, non-small cell lung cancer, bladder cancer, cervical, vulvar or anal cancer, esophageal cancer, metastatic head and neck cancer, liver cancer, lymphoma, multiple myeloma, ovarian cancer, pancreatic cancer, acute myeloid leukemia, Kaposi sarcoma.

The A_{2B} adenosine receptor subtype (Feoktistov, et al. I. Pharmacol. Rev. 1997, 49, 381-402) has been identified in a variety of human and murine tissues and is involved in the regulation of vascular tone, smooth muscle growth, angiogenesis, hepatic glucose production, bowel movement, intestinal secretion, and mast cell degranulation.

A_{2B} receptors have been implicated in mast cell activation and asthma, control of vascular tone, cardiac myocyte contractility, cell growth and gene expression, vasodilation, regulation of cell growth, intestinal function, and modulation of neurosecretion (Pharmacological Reviews, 2003, 49, 4).

A_{2B} receptors modulate mast cell function. Adenosine activates adenylate cyclase and protein kinase C and potentiates stimulated mediator release in mouse bone marrow derived mast cells. Activation of A_{2B} receptors in HMC-1 augments IL-8 release and potentiates PMA-induced secretion of IL-8. Thus, adenosine would contribute to the asthmatic response by acting on the mast cell to enhance the release of proinflammatory mediators. *(*Pulmonary Pharmacology & Therapeutics 1999, 12, 111-114). In COPD, transformation of pulmonary fibroblasts into myofibroblasts is considered a major mechanism. Activation of the A_{2B} AR is involved in this process. Selective A_{2B} antagonists are expected to have beneficial effect on pulmonary fibrosis (Curr. Drug Targets, 2006, 7, 699-706; Am. J. Resper. Cell. Mol. Biol., 2005, 32, 228). A_{2B} antagonists can be used as wound healing agents. Activation of the A_{2B} AR promotes angiogenesis by increasing the release of angiogenic factors and A_{2B} antagonists are useful to block angiogenesis (Circ. Res., 2002, 90, 531-538). A_{2B} AR may be involved in the inhibition cardiac fibroblast (CF) proliferation (Am.J. Physiol. Heart Circ. Physiol., 2004, 287, H2478-H2486). Adenosine stimulates Cl-secretion in the intestinal epithelia pointing towards a possible treatment for cystic fibrosis patients with CFTR mutation (Am. J. Respir. Cell Mol. Biol., 2008, 39, 190-197). High affinity A_{2B} antagonists are effective in hot plate model suggestive of the role of A_{2B} in nociception and can be used as potential analgesic agents (The J. of Pharmacol. and Exp. Ther., 2004, 308, 358-366).

A_{2B} receptor is involved in release of IL-6. Increasing evidence suggests that IL-6 plays a role in Alzheimer's disease in the context of inflammatory process associated with disease. Hence A_{2B} receptor antagonist might be useful for Alzheimer's disease.

The A_{2B} ARs are involved in the stimulation of nitric oxide production during Na⁺-linked glucose or glutamine absorption. They are involved in glucose production in hepatocytes upon agonist stimulation. A_{2B}-receptor antagonists showed an anti-diabetic potential mainly by increasing plasma insulin levels under conditions when the adenosine tonus was elevated in-vivo and increased insulin release in-vitro (J Pharm. Pharmacol. 2006 Dec; 58(12):1639-45). Thus, A_{2B} antagonists may serve as a novel target for the treatment of this metabolic disease.

It has been demonstrated that adenosine activation of the A_{2B} adenosine receptor increase cAMP accumulation, cell proliferation and VEGF expression in human retinal endothelial cells. Activation of A_{2B}AdoR increased vascular endothelial cell growth factor mRNA and protein expression in human retinal endothelial cells. Adenosine also has a synergistic effect with VEGF on retinal endothelial cell proliferation and capillary morphogenesis in vitro. Such activity is necessary in healing wounds, but the hyperproliferation of endothelial cells promotes diabetic retinopathy. Also, an undesirable increase in blood vessels occurs in neoplasia. Accordingly, inhibition of binding of adenosine to A_{2B} receptors in the endothelium will alleviate or prevent hypervasculation, thus preventing retinopathy and inhibiting tumor formation.

Adenosine generation in tumor microenvironment is an active metabolic mechanism used by cancer cells to avoid anti-tumor immunosurveillance and increase metastasis. Ectonucleotidase CD73 and CD39 (highly expressed on tumor cells and stromal cells) convert ATP released by dying tumor cells to adenosine. A_{2B} receptors are expressed at low levels on multiple cell types under normal conditions, but significantly upregulated under hypoxic conditions that prevail in the tumor microenvironment. Activation of A_{2B} receptors promotes angiogenesis and causes T cell and myeloid derived suppressor cell (MDSC) mediated immunosuppression in the tumor microenvironment. A_{2B} antagonists could induce anti-tumoral responses in multiple types of cancer when used as stand alone or in combination with existing immunotherapies or radiotherapy or chemotherapy. Cancers that could benefit from A_{2B} antagonist therapy include melanoma, triple negative breast cancer, colon cancer, colorectal cancer, lung cancer, prostate cancer, renal cell cancer, non-small cell lung cancer, bladder cancer, cervical, vulvar or anal cancer, esophageal cancer, metastatic head and neck cancer, liver cancer, lymphoma, multiple myeloma, ovarian cancer, pancreatic cancer, acute myeloid leukemia, Kaposi sarcoma.

Adenosine A_{2B} receptors are ubiquitous and regulate multiple biological activities. For example, adenosine binds to A_{2B} receptors on endothelial cells, thereby stimulating angiogenesis. Adenosine also regulates the growth of smooth muscle cell populations in blood vessels. Adenosine stimulates A_{2B} receptors on mast cells, thus modulating Type I hypersensitivity reactions. Adenosine also stimulates gastrosecretory activity by ligation with A_{2B} in the intestine.

While many of these biological effects of adenosine are necessary to maintain normal tissue homeostasis, under certain physiological changes it is desirable to modulate its effects. For example, the binding of A_{2B} receptors stimulates angiogenesis by promoting the growth of endothelial cells. Such activity is necessary in healing wounds, but the hyperproliferation of endothelial cells promotes diabetic retinopathy. Also, an undesirable increase in blood vessels occurs in neoplasia. Accordingly, inhibition of the binding of adenosine to A_{2B} receptors in the endothelium will alleviate or prevent hypervasculation, thus preventing retinopathy and inhibibiting tumor formation.

A_{2B} receptors are found in the colon in the basolateral domains of intestinal epithelial cells, and when acted upon by the appropriate ligand act to increase chloride secretion, thus causing diarrhea, which is a common and potentially fatal complication of infectious diseases such as cholera and typhus. A_{2B} antagonists can therefore be used to block intestinal chloride secretion and are thus useful in the treatment of inflammatory gastrointestinal tract disorders, including diarrhea. Another adverse biological effect of adenosine acting at the A_{2B} receptor is the overstimulation of cerebral IL-6, a cytokine associated with dementias and Alzheimer's disease. Dipti Vijayan et al. (Nature Reviews (2017), 17(12), 709 - 724) reports the effects of the A2A receptor antagonist CPI-444 in combination with an anti-PD-L1 antibody in triple negative breast cancer and colorectal cancer.

Accordingly, it is desired to provide compounds that are potent A_{2A}/A_{2B} antagonists (i.e., compounds that inhibit the A_{2A}/A_{2B} adenosine receptor), fully or partially selective for the A_{2A}/A_{2B} receptor, useful in the treatment of various disease states related to modulation of the A_{2A}/A_{2B} receptor, for example cancer.

### SUMMARY OF THE INVENTION

In an aspect of the invention there is provided a pharmaceutical composition comprising 5-amino-3-[2-[4-[2-fluoro-4-(2-methoxyethoxy)phenyl]piperazin-1-yl]ethyl]-8-(2-furyl)-1-methyl-[1,2,4]triazolo[5,1-f]purin-2-one **(32)** or a pharmaceutically acceptable salt thereof for use in the treatment of a condition or disorder selected from breast cancer and colon cancer.

### DETAILED DESCRIPTION

It is understood that the present invention does not relate to methods for treatment of the human or animal body by surgery or therapy, or to diagnostic methods practiced on the human or animal body.

Those skilled in the art will be aware that the present disclosure is subject to variations and modifications other than those specifically described provided that they are within the scope of the appended claims. It is to be understood that the present disclosure includes all such variations and modifications. The disclosure also includes all such steps, features, compositions and compounds referred to or indicated in this specification, individually or collectively, and any and all combinations of any or more of such steps or features, provided that they are within the scope of the appended claims.

### Definitions

For convenience, before further description of the present disclosure, certain terms employed in the specification, and examples are collected here. These definitions should be read in the light of the remainder of the disclosure and understood as by a person of skill in the art. The terms used herein have the meanings recognized and known to those of skill in the art, however, for convenience and completeness, particular terms and their meanings are set forth below.

The articles "a", "an" and "the" are used to refer to one or to more than one (i.e., to at least one) of the grammatical object of the article.

Throughout the description and the claims which follow, unless the context requires otherwise, the word "comprise", and variations such as "comprises" and "comprising", will be understood to imply the inclusion of a stated integer or step or group of integers but not to the exclusion of any other integer or step or group of integers or steps.

The term "including" is used to mean "including but not limited to". "Including" and "including but not limited to" are used interchangeably.

The term "disorder or condition ameliorated by the inhibition of the A_{2A} receptor" will be understood by those skilled in the art to include: cancer such as prostate, rectal, renal, ovarian, endometrial, thyroid, pancreatic, particularly breast, colon, bladder, brain, glia, melanoma, pineal gland and, more particularly, lung cancer (e.g. Lewis lung carcinoma).

The term "therapeutically effective dose" means an amount of a compound or composition which is sufficient enough to significantly and positively modify the symptoms and/or conditions to be treated (e.g., provide a positive clinical response). The effective amount of an active ingredient for use in a pharmaceutical composition will vary with the particular condition being treated, the severity of the condition, the duration of the treatment, the nature of concurrent therapy, the particular active ingredient(s) being employed, the particular pharmaceutically-acceptable excipient(s)/carrier(s) utilized, the route of administration, and like factors within the knowledge and expertise of the attending physician.

The phrase "pharmaceutically acceptable excipient" refers to compounds or compositions that are physiologically tolerable and do not typically produce allergic or similar untoward reactions, including but not limited to gastric upset or dizziness when administered to mammal.

The term "pharmaceutically acceptable salt" embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic or p-toluenesulphonic acid. Pharmaceutically acceptable bases include alkali metal (e.g. sodium or potassium) and alkali earth metal (e.g. calcium or magnesium) hydroxides and organic bases, for example alkyl amines, arylalkyl amines and heterocyclic amines.

Other preferred salts according to the invention are quaternary ammonium compounds wherein an equivalent of an anion (X-) is associated with the positive charge of the N atom. X- may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and p-toluenesulphonate. X- is preferably an anion selected from chloride, bromide, iodide, sulphate, nitrate, acetate, maleate, oxalate, succinate or trifluoroacetate. More preferably X- is chloride, bromide, trifluoroacetate or methanesulphonate.

According to the claimed invention, there is provided a pharmaceutical composition comprising 5-amino-3-[2-[4-[2-fluoro-4-(2-methoxyethoxy)phenyl]piperazin-1-yl]ethyl]-8-(2-furyl)-1-methyl-[1,2,4]triazolo[5,1-f]purin-2-one **(32)** or a pharmaceutically acceptable salt thereof for use in the treatment of a condition or disorder selected from breast cancer and colon cancer.

The use may include treatment of a disease or condition in a mammal that is amenable to treatment with an A_{2A}/A_{2B} receptor antagonist, the use comprising: administering to a mammal in need thereof a therapeutically effective dose of the pharmaceutical composition for use as defined in the appended claims.

Also forming part of the disclosure is a pharmaceutical composition comprising compound 32, in combination with at least one PD-L1 antibody, for use in the treatment of a condition or disorder selected from breast cancer and colon cancer.

The pharmaceutical composition of the present invention is for use in the treatment of a condition or disorder ameliorated by inhibition of the A_{2A}/A_{2B} receptor, wherein the condition or disorder is selected from breast cancer and colon cancer.

### Example 1

### Synthesis of Compound 32

Compound 32 was synthesized as per the procedure mentioned in WO2012038980. Pharmaceutically acceptable salts of the compound may be obtained as per procedures reported in literature.

### Example 2

### Synthesis of Compound 335

Phosphoric acid mono-{2-cyano-6-oxo-1-propyl-8-[1-(3-trifluoromethyl-benzyl)-1H-pyrazol-4-yl]-1,6-dihydro-purin-7-ylmethyl} ester, compound 335, was synthesized as per the procedures mentioned in WO2012035548. Pharmaceutically acceptable salts of the compound may be obtained as per procedures reported in literature.

The compounds of the disclosure may be prepared by a variety of methods, including standard synthetic chemistry. Any previously defined variable will continue to have the previously defined meaning unless otherwise indicated. Illustrative general synthetic methods are set out in the schemes and can be readily adapted to prepare other compounds of the disclosure.

### Example 3

### Biological Assay

### Tumor suppression activity of Adenosine A_{2A} and A_{2B} Antagonists in xenograft models of cancer

General Protocol: 6-8 weeks old BALB/c mice were acclimated and on Day 1 of the study, the mice were injected with 50 µL of medium containing 5 X10⁴ 4T1 cells (breast cancer) or 5 X 10⁵ CT26 cells (colon cancer). On day 8 or 9, the mice were segregated into different groups and treated orally with either vehicle (1% Tween-80+ 0.5% Carboxymethylcellulose in water) or test compound [(Compound 32 5-Amino-3-[2-[4-[2-fluoro-4-(2-methoxyethoxy)phenyl]piperazin-1-yl]ethyl]-8-(2-furyl)-1-methyl-[1,2,4]triazolo[5,1-f]purin-2-one **(32) &** Phosphoric acid mono-{2-cyano-6-oxo-1-propyl-8-[1-(3-trifluoromethyl-benzyl)-1H-pyrazol-4-yl]-1,6-dihydro-purin-7-ylmethyl} ester **(335)]** in vehicle. The treatment was BID for 22 days (colon cancer) or 28 days (breast cancer). At the end of the study, the tumor volume was measured as (length X breadth)/2. Data were presented as % reduction compared to the tumor volume in vehicle treated animals. The use of Compound 335 represents a comparative example.

| **4T1 Breast Cancer Model** | |
|---|---|
| Treatment Groups (n=12) | % decrease in Tumor growth *vs*. vehicle On Day 28 |
| Compound 32, 1 mg/kg, *PO, **BID | 48.30 |
| Compound 335, 3 mg/kg, *PO, **BID | 46.40 |

| **CT26 Colon Cancer Model** | |
|---|---|
| Treatment Groups (n=12) | % decrease in Tumor growth *vs*. vehicle On Day 22 |
| Compound 32, 1mg/kg, *PO, **BID | 54.40 |
| Compound 335, 3mg/kg, *PO, **BID | 23.90 |

| | |
|---|---|
| *PO= per os, i.e., by mouth; **BID= bis in die, i.e., twice a day | |

## Claims

1. A pharmaceutical composition comprising 5-amino-3-[2-[4-[2-fluoro-4-(2-methoxyethoxy)phenyl]piperazin-1-yl]ethyl]-8-(2-furyl)-1-methyl-[1,2,4]triazolo[5,1-f]purin-2-one **(32)** or a pharmaceutically acceptable salt thereof for use in the treatment of a condition or disorder selected from breast cancer and colon cancer.

2. The pharmaceutical composition for use of claim 1, further comprising at least one PD-L1 antibody.

## Patentansprüche

1. Pharmazeutische Zusammensetzung, die 5-Amino-3-[2-[4-[2-fluor-4-(2-methoxyethoxy)phenyl]piperazin-1-yl]ethyl]-8-(2-furyl)-1-methyl-[1,2,4]triazolo[5,1-f]purin-2-on **(32)** oder ein pharmazeutisch annehmbares Salz davon zur Verwendung bei der Behandlung einer Erkrankung oder Störung, ausgewählt aus Brustkrebs und Kolonkarzinom, umfasst.

2. Pharmazeutische Zusammensetzung zur Verwendung nach Anspruch 1, weiter umfassend mindestens einen PD-L1-Antikörper.

## Revendications

1. Composition pharmaceutique comprenant un 5-amino-3-[2-[4-[2-fluoro-4-(2-méthoxyéthoxy)phényl]pipérazin-1-yl]éthyl]-8-(2-furyl)-1-méthyl-[1,2,4]triazolo[5,1-f]purin-2-one **(32)** ou un sel pharmaceutiquement acceptable de celui-ci pour une utilisation dans le traitement d'une affection ou d'un trouble sélectionné parmi le cancer du sein et le cancer du côlon.

2. Composition pharmaceutique pour une utilisation selon la revendication 1, comprenant en outre au moins un anticorps PD-L1.
